# EUROPEAN PATENT APPLICATION

(11) **EP 4 207 208 A1**
(43) Date of publication of application: **05.07.2023**
(21) Application number: 21397510.5
(22) Date of filing: 29.12.2021
(51) Int. Cl.: G16H 10/40

(54) **A METHOD, A SYSTEM, AND A WEARABLE ELECTRONIC DEVICE**

(71) Applicant: Sartorius Biohit Liquid Handling Oy, 00880 Helsinki (FI)
(72) Inventor: KANKAANPÄÄ, Pasi, 00880 Helsinki (FI); HINTIKKA, Ville, 00880 Helsinki (FI); SALIVAARA, Kari, 00880 Helsinki (FI)
(74) Representative: Laine IP Oy

(57) **Abstract**

According to an example aspect of the present invention, there is provided a method, comprising: providing a wearable electronic device that is configured to be worn by a user of a laboratory device; by means of the wearable device, retrieving user data that is specific to said user; and on the basis of the retrieved user data, providing a functionality for control of the laboratory device.

## Description

### FIELD

The present application relates to a method of controlling or operating a laboratory device, particularly a liquid handling device.

### BACKGROUND

Electronic pipettes are widely used for liquid handling in tasks involving complex chemical analyses. An electronic pipette is controlled via its user interface, which comprises buttons and a display screen integrated to the handle of the pipette. The size and shape of the handle imposes natural restrictions to the design and versatility of the user interface, which further may have negative consequences on the performance of the pipette.

In laboratories, user identification data is typically not automatically available or it is unreliable. As a result, error tracking may be incomplete or difficult, and personalization of laboratory device user interfaces cumbersome and may require manual user input.

The invention intends to solve at least some of the above discussed problems.

### SUMMARY OF THE INVENTION

The invention is defined by the features of the independent claims. Some specific embodiments are defined in the dependent claims.

According to a first aspect of the present invention, there is provided a method, comprising: providing a wearable electronic device that is configured to be worn by a user of a laboratory device; by means of the wearable device, retrieving user data that is specific to said user; and on the basis of the retrieved user data, providing a functionality for control of the laboratory device.

Various embodiments of the first aspect may comprise one or more features from the following bulleted list:
- Said wearable electronic device is configured to be worn around a body part of the user.
- Said wearable electronic device comprises a smart watch, a smart bracelet, a smart ring, a smart necklace or smart glasses, preferably a smart watch.
- Said laboratory device is a liquid handling device or a liquid dosing device, such as a hand-held electronic pipette or an automated liquid handling or dosing device.
- Said laboratory device is a pipette, such as a single-channel pipette or a multichannel pipette.
- Said laboratory device is a mechanical pipette or a semi-mechanical pipette or an electronic pipette.
- Said laboratory device is a laboratory balance.
- Said laboratory device is an automated sample handling or analysis device.
- Said laboratory device is located in a sterile environment or space, such as in a sterile laminar flow hood or a sterile room.
- Said wearable device is located outside said sterile space.
- The laboratory device does not comprise any physical buttons.
- The laboratory device does not comprise any display.
- The laboratory device is sterilisable, preferably in its entirety.
- The wearable electronic device and the laboratory device are connected or connectable to each other by a wireless connection.
- Said user data comprises user identity data.
- Said user data comprises usage history data specific to said user.
- Said user data comprises user location data, such as distance of the wearable device worn by the user from the laboratory device.
- Said functionality is executable either manually, for example by selecting or activating, by the user or automatically by the laboratory device.
- Said executable functionality is provided for use or execution either in the wearable electronic device and/or in the laboratory device.
- Said executable functionality is provided in the laboratory device.
- Said functionality comprises automated locking or automated unlocking of the laboratory device.
- Said functionality, such as locking of the laboratory device, is executed automatically when said user data satisfies a pre-determined criterion, such as when the distance between the user and the laboratory device is less than a predetermined minimum distance.
- Said executable functionality is provided in the wearable electronic device.
- Said functionality comprises remote control of at least one function of the laboratory device.
- Said remote control of the laboratory device is provided in the wearable electronic device via a user interface in the wearable electronic device.
- Said executable functionality is provided in the wearable electronic device, and said functionality comprises providing a user-customized user interface of the laboratory device.
- Said user-customized user interface of the laboratory device is provided in the wearable device via input and/or output means, such as a display and/or buttons of the wearable electronic device.
- Said user interface or said user-customized user interface of the laboratory device is a user motion controlled user interface of the laboratory device.
- If no user data can be retrieved, no functionalities for control of the laboratory device are provided, and preferably use of the laboratory device is disabled fully or partially, for example by locking the laboratory device or at least one or more of its operational modes.
- The method comprises retrieving environmental status data that is specific to the laboratory device, such as temperature data, air humidity data, fleet management data, or data related to an atmosphere.
- On the basis of the retrieved user data and the retrieved environmental status data, providing a functionality for control of the laboratory device.

According to a second aspect of the present invention, there is provided a laboratory device, configured to be used in a method according to the first aspect.

According to a third aspect of the present invention, there is provided a wearable electronic device, configured to be used in a method according to the first aspect.

According to a fourth aspect of the present invention, there is provided a system, comprising a wearable electronic device and a laboratory device connected to each other by a wireless connection, wherein said system is configured to be used in a method according to the first aspect.

According to a fifth aspect of the present invention, there is provided use of a wearable electronic device, such as a smart watch, for providing user data which is specific to a user of a laboratory device, wherein on the basis of said user data, a functionality for control of the laboratory device is provided.

According to a sixth aspect of the present invention, there is provided a wearable electronic device, which is connectable to a laboratory device and configured to be worn by a user of the laboratory device, the wearable electronic device comprising: means for retrieving user data that is specific to said user; and means for providing, on the basis of the retrieved user data, a user-specific user interface including at least one functionality for control of the laboratory device.

### EMBODIMENTS

It has been surprisingly observed that by means of an electronic wearable device it may be possible to provide to a user novel, effective and safe ways to control laboratory devices.

The invention concerns a method, comprising providing a wearable electronic device that is configured to be worn by a user of a laboratory device; by means of the wearable device, retrieving user data that is specific to said user; and on the basis of the retrieved user data, providing a functionality for control of the laboratory device.

In the following, any reference to "wearable device" typically refers to "wearable electronic device".

Typically said wearable electronic device is configured to be worn around a body part of the user.

Preferably, the wearable device is detachably worn by a user on or around his body or body part, for example around a wrist or a finger. The hands of the user are left free in the sense that the user can freely use his/her both hands in order to for example carry out a liquid handling task.

For example, the user may wear the wearable device in the hand with which he is holding the pipette. Alternatively the user may choose to wear the wearable device in another hand.

In an embodiment, the user may wear the wearable device so that he is able to observe its display.

In an embodiment, the user may wear the wearable device so that he is able to use a user interface integrated to the wearable device for example by touching, by voice control or by gesture control.

In some embodiments, the wearable device may be operated without touching it and even via a user interface of an external electronic device, to reduce crosscontamination.

In some embodiments, the wearable device is attachable to a user's body so that it is in constant and direct contact with the user's skin. Such skin contact can be utilized for example for the purpose of detecting the user's heart rate.

In an embodiment, the wearable device may be worn around or on a movable (in a use situation) body part, such as a hand, a wrist or a finger.

In an embodiment, the wearable device may be worn around or on a substantially stationary (in a use situation) body part, such as a neck or a foot.

A use situation may refer to use of a laboratory device, such as a pipetting event executed by a user.

In some embodiments, the wearable device may be worn around or on a body part that, in a use situation, is located or can be placed near the laboratory device, such as a finger or a wrist.

In some embodiments, the wearable device may be worn around or on a body part that, in a use situation, faces or points towards the laboratory device, for example for the purpose a recording a video, taking a photograph or establishing a data connection. An exemplary embodiment is smart glasses that are worn on a user's nose.

Alternatively, the wearable device itself may be turnable so that the user may orient the worn device to a desired configuration in a three-dimensional space.

For example, said wearable electronic device may comprise a smart watch, a smart bracelet, a smart ring, a smart necklace or smart glasses. Particularly preferred is a smart watch.

The wearable device may comprise a user interface. The user interface is configured to input and/or output data. Output means may comprise a display or a screen for presenting data to a user. Input means may comprise keys, buttons, a microphone and/or a camera. A touch display may be used for inputting and outputting data. An accelerometer may be used for identifying orientation of the wearable device. The wearable device may comprise a memory, which may store data and/or executable instructions; a processor, a controller, voice control means, gesture control means, control software, connection means for providing wireless and/or wired connection. Wireless connection may be implemented via wireless reader, radio frequency (RF) tag, infrared or Bluetooth connection. Connection means may enable both sending and receiving data via the established connection. Data may comprise instructions, control data, signalling, variables, or any other data.

Said laboratory device may be a liquid handling or liquid dosing device, such as a hand-held electronic pipette or an automated liquid handling or dosing device. In an embodiment, the laboratory is device is any electronic laboratory device that is configured to be controlled or operated either via a user interface integrated to the laboratory device itself and/or via a remotely located user interface.

Said laboratory device may be any electronic or semi-electronic laboratory device, such as a pipette, a dispenser, a burette, a liquid handling station, a laboratory balance, a sample analysator, a filtration device, a bioreactor, a fermenter, a flow cytometer, a live cell imaging and/or analysis device, a chromatography device or system, a protein analysator or a water purification or dispensing device or system.

In one embodiment, the laboratory device is an electronic liquid handling device.

In one embodiment, the laboratory device is an electronic laboratory balance.

In one embodiment, the laboratory device is a mechanical or semi-mechanical pipette capable of wireless communication with the wearable electronic device. Preferably, on the basis of the retrieved user data, there is provided a functionality for control of one or more electronic functions of the mechanical or semi-mechanical pipette.

In an embodiment, said laboratory device is located in a clean or sterile or sterilisable environment, such as in a sterile laminar flow hood or a sterile room or a clean room or a sterile chamber. The wearable device enables control and operation of the laboratory device remotely, at a selected location different from the location of the laboratory device.

The wearable device is advantageous for example in a clean room environment or when using a laminar flow hood or other clean working cabinet, into which it is not possible to bring a user's tablet or mobile phone. The user wearing the wearable device may even stay outside the clean room environment or at least the user may keep a longer distance to the laboratory device than what would be possible without the wearable device, and still he may control the laboratory device. Alternatively the user may enter the clean room environment but with less equipment, such as a laptop.

For example, in the case of a laminar flow hood, it is typically not possible to place a laptop or any other smart device in the hood, because the workspace inside the hood is limited and the items usually need to be sterilized before introducing them to the hood. Therefore, any use of a laptop in the course of work inside a laminar flow hood is cumbersome or even impossible and necessitates interruption of work. Via a user interface of a wearable device the user may perform functions, such as make changes to protocols or settings of a laboratory device, without having to rely on a laptop or computer outside the hood.

Reliable user identification in the context of pipetting may be crucial in the following situations: strict traceability requirements, hospital environments. By means of a wearable device it can be better ensured that user identification data is reliable.

The wearable electronic device and the laboratory device are preferably connected to each other by a wireless connection. Alternatively a wired connection may be used. The connection may enable transmission of data and commands enabling control of the laboratory device in accordance with some embodiments. The connection may enable receiving data, like measured results and settings, of the laboratory device.

The wearable device and the laboratory device may connect with each other automatically when they are sufficiently close to each other and both turned on. Alternatively, the user may connect the devices by a command that activates or creates the connection.

The wireless connection may be implemented by means of an RFID (radio frequency identification) or NFC (near-field communication) tag and reader. Preferably the working distance is short, such as less than 0.5 m. A short working distance is advantageous as it limits the functions of the wearable device for use only in a close proximity of an external laboratory device.

In an embodiment, the wearable device is dedicated to the user who is wearing the wearable device. The wearable device is then capable of providing user identity data to external devices, such as a pipette. The wearable device may receive the user identity data by various means, for example from a log file accessible only when the wearable device is being worn or active and/or when a user pulse or other presence data is detected by the wearable device.

The wearable device may be configured for use with only particular types of laboratory devices or a determined, single laboratory device. Additionally the wearable device may be dedicated to and identifies the user who puts on the wearable device and wears it.

In some embodiments, the user wearing the wearable device is identified, for example by means of his heart rate or any other suitable measurable physiological quantity or parameter. Alternatively, the user may input identification data to the wearable device, for example biometric identification data (fingerprint recognition, iris recognition, face recognition) or alphanumeric identification data.

The wearable device provides clear advantages in comparison to merely portable devices such as tablets or smart phones: identity, location and/or presence of the user can be verified more reliably as there is a firm link between the wearable device and the user wearing the wearable device.

In an embodiment, the wearable device is both wearable and portable. Portability typically comprises light weight of the wearable device and ability of the user to carry the wearable device when the user is moving, such as walking.

In some embodiments, the wearable device is used for retrieving user data that relates to the user that is currently wearing the wearable device. Advantageously, the user data is retrieved when the user is preparing for or in the process of operating a laboratory device. Retrieval of the user data typically forms the basis for providing functionalities for control of the laboratory device. Said providing may involve making available new or modified features or functionalities in the wearable device or in the laboratory device itself.

Said user data may comprise user identity data or usage history data specific to said user or user location data, such as distance of the user from the laboratory device. Usage history data may relate log or history data collected and saved during previous use of the laboratory device by the wearable device and/or the identified user. User location data may be based on location of the wearable device. Distance of the user from the laboratory device may correspond to the distance of the laboratory device from the wearable device.

Thus, said user data may relate to the identity and/or characteristics of the user.

Said user data may also relate to both the user and the laboratory device, such as to a user-specific manner of operating the laboratory device. Advantageously at least some embodiments enable taking account of user-specific settings, preferences or restrictions related to operating the laboratory device. These settings, preferences or restrictions are typically reflected or comprised in the user data retrieved by the wearable device.

The provided new feature or functionality is preferably suitable or usable or executable for control of the laboratory device. In some embodiments, the use or activation or execution of the provided functionality has an effect on the operation of the laboratory device.

Said functionality may be executable or usable either manually, such as when desired or needed, by the user or automatically by the laboratory device.

Said executable functionality may be provided either in the wearable electronic device and/or in the laboratory device.

The means. such as a processor, a memory and software, for execution of the functionality may reside in the wearable device and/or in the laboratory device.

Based on the user data, the wearable device may send a command or instruction to the laboratory device which then may provide an executable functionality. Said executable functionality may be executed automatically or it may be utilized or executed by the user when controlling the laboratory device.

For example, said executable functionality may be provided in a library or memory in the wearable device, from which library the user may select the functionality for use. The library is preferably opened for use by the user on the basis of the detected or retrieved user data.

For example, said functionality may comprise automated locking or automated unlocking of the laboratory device, for example a part of or all of the functional features of the laboratory device, on the basis of the content of the user data, such as user location data.

For example, said functionality may comprise means for manual locking or manual unlocking of the laboratory device, for example a part of or all of the functional features of the laboratory device, such as via a user interface that is provided for the user on the basis of the content of the user data. The user may provide an input, such as a selection or a setting, via said user interface to lock or unlock the laboratory device.

In some embodiments, the user interface that is available in a laboratory device, such as a liquid handling device, may be tailored or modified on the basis of the user data. The user interface may comprise pipetting modes. In an example, the pipetting modes that are available in a liquid handling device may be tailored on the basis of the user data. In this case the provided new functionality comprises the user-tailored set of pipetting modes.

Said functionality, such as locking or unlocking of the laboratory device or some of its operating modes, may be executed automatically when said user data satisfies a pre-determined criterion, such as when the distance between the user and the laboratory device is less than a pre-determined minimum distance. Typically such automated locking or unlocking is carried out by means of a software and a processor in the laboratory device.

In an embodiment, said executable functionality is provided in the wearable electronic device, and said functionality may comprise remote control of at least one function of the laboratory device. The remote control of the laboratory device may be provided in the wearable electronic device via a user interface that is integrated to the wearable electronic device.

In an embodiment, said executable functionality is provided in the wearable electronic device, and said functionality comprises providing a user-customized user interface for control of the laboratory device or at least some of its functions or operating modes.

Said user-customized user interface of the laboratory device may be provided in the wearable device via a user interface, like a display and/or buttons, integrated in the wearable electronic device. For example, the user interface, like a display and/or buttons, may be the same display and buttons that form part of the user interface for control of the wearable device itself, such as the smart watch itself. Switching between these two user interfaces may be carried out manually by the user. The user-customized user interface for controlling the laboratory device may be activated in the wearable device, when distance between the two devices, or distance between the user and the laboratory device, is below a predetermined threshold.

In addition to or instead of said display and buttons, the user interface for remote control of the laboratory device may be realized by means of one or more input means, like sensors integrated in the wearable device, such as a motion sensor, a voice sensor and alike, to enable remote voice and/or motion control of the laboratory device. In addition or alternatively, the wearable device may comprise a camera or a scanner.

In an embodiment, said user interface or said user-customized user interface of the laboratory device is a user motion controlled user interface of the laboratory device. In this case it is preferred that the wearable device is worn on or around a hand of the user or a part thereof.

In case of voice control, the wearable device is preferably worn in a close proximity to the user's mouth, such as a smart necklace.

### User interface

In an embodiment, the wearable device comprises an enhanced user interface in comparison to the laboratory device, such as a liquid handling device, with which it communicates in order to facilitate remote operating or controlling of the laboratory device. Such an enhanced user interface may comprise gesture control, such as a radar chip, which typically cannot be implemented to a hand-held laboratory device. For example, in a pipette, forming gestures during pipetting is practically inconvenient.

In an embodiment, the user interface of the wearable device is adaptable or adapted or altered for example as a function of user identity, location, liquid handling device, and/or liquid handling task. In this way the usability of the laboratory device can be improved and accommodated to the needs of the user. A laboratory device may be personalized in a use situation, thus reducing the need to provide each user with his own laboratory equipment, such as pipettes. For example, the user interface of the wearable device may only comprise a relatively small display screen and a limited amount of buttons, if the function and intended use of the display and the buttons is altered as a function of the laboratory device in question and its use. The buttons may be for example virtual buttons on a touch display or real buttons.

In an embodiment, the wearable device comprises a user interface providing substantially the same input and output functionalities as the normal user interface integrated in a laboratory device, such as a liquid handling device with which it communicates in order to enable remote operating of the liquid handling device.

In embodiments involving sterile environments, the laboratory device, such as a liquid handling device, might lack any physical control buttons and/or display means and preferably exhibit a smooth and easily sterilisable or cleanable surface. In an embodiment, the laboratory device is sterilisable in its entirety and only has sterilisable components. In this way, complete purification of the laboratory device becomes possible and the risk of contamination reduced.

Preferably, the user may control and/or operate the laboratory device, such as a pipette, via the user interface of the wearable communication device. The instructions and data transmitted from the wearable device to the pipette may be accompanied by user identification data, which the pipette may utilize and for example attach to a log file generated in the course of executing a pipetting task in accordance with the received instructions. Such a log file may be generated by the liquid handing device and stored in its memory. In one example, user specific use history data is generated and stored, for example in a memory of the liquid handling device.

Remote operation of a pipette is preferred in situations in which the user is not capable of for example entering the space in which the pipette is located or the user is handicapped or the user is in need of an improved pipette user interface or the user prefers not to touch the pipette.

In some embodiments, the wearable device is configured to provide an alternative user input method, such as a technologically advanced user input method, for the use or control of a laboratory device. This is advantageous particularly in cases where such an advanced or alternative user input method is not compatible for integration into said laboratory device and/or in cases where such user input method is only required in certain user-specific situations.

The invention also concerns a system comprising an electronic wearable device and a laboratory device.

Typically, the system comprises means for establishing a wireless connection between the wearable device and the laboratory device. Via the wireless connection, it may be possible to transmit said user data and/or laboratory device data between the wearable device and the laboratory device. The laboratory device data may comprise laboratory device status data.

In an embodiment, the wearable device comprises means for, on the basis of at least the laboratory device data received from the laboratory device, preferably in combination with user data, making available to the user a functionality via the wearable device, wherein said functionality is configured to be used by the user for or during controlling the laboratory device while wearing the wearable device.

In another embodiment, the laboratory device comprises means for, on the basis of the user data received from the wearable device, making available to the user a functionality in the laboratory device or disabling from the user a functionality in the laboratory device.

Various further features may be realized by means of the wearable device.

In some embodiments, the wearable device is configured to receive status data from a laboratory device, such as a liquid handling device or a pipette. The wearable device may convey it further to the user of the wearable device for example via its own integrated display or other display means. Such status data may include any of the following: instructions related to performing of a pipetting task, alarm signals related to the status (e.g. calibration status, battery status) of a liquid handling device, pipette location and availability data, protocols available for use in a liquid handling device, information related to eventual mistakes or deviations in the course of recent pipetting events, instructions for compensating or rectifying such errors or deviations. The retrieved laboratory device status data may be used in combination with the retrieved user data as a basis for providing a particular functionality for control of the laboratory device

In some embodiments, the wearable device is configured to receive or retrieve environmental status data that is specific to the laboratory device or its environment, such as temperature data, air humidity data, fleet management data, or data related to an atmosphere. The environmental status data may correspond to status data inside a laminar flow hood. The wearable device may convey the environmental status data further for the user of the wearable device for example via its own integrated display or other display means. The environmental status data may be received from one or more sensors located in the proximity of the laboratory device or integrated to its structure or integrated to a laboratory space, room or facility in which the laboratory device is located. The retrieved environmental status data may be used in combination with the retrieved user data as a basis for providing a particular functionality for control of the laboratory device.

In an embodiment, the wearable device may retrieve and utilize data that is external to the laboratory device, such as a pipette, and not retrievable by the laboratory device itself. Such external data may be used for the benefit of improving accuracy and reliability of the liquid handling task. The external data may be used in combination with user data as a basis for providing a particular functionality for control of the laboratory device.

In some embodiments, the wearable device is configured to function as a data exchange hub between multiple laboratory devices. Laboratory devices that are connected to the wearable device advantageously include one or more of the following: a liquid handling device, an altimeter, a barometer, a temperature sensor, an air humidity sensor. Multiple devices of each of the listed categories may be connected to each other.

In some embodiments, the wearable device is configured to receive input, commands or data from the user. Such input, command or data may include: user identification data, user-specific settings of a laboratory device, personal settings or control input for the wearable device itself and/or for a laboratory device.

The invention also concerns a system comprising a wearable electronic device and a laboratory device connected to each other by a wireless connection.

The invention further concerns use of a wearable electronic device, such as a smart watch, for providing user data which is specific to a user of a laboratory device. Said user is wearing the wearable device. On the basis of said user data, a functionality for control of the laboratory device is provided or made available to the user.

### Examples

### Example 1: Control of an automated laboratory device, such as an automated liquid handling robot

For control of an automated liquid handling robot or other automated laboratory device, the wearable device may be a smart watch or a smart glove.

The wearable device may provide safety features in connection with an automated liquid handling robot. For example, the functionality that is provided on the basis of user data, such as user proximity, may comprise automated locking of the robot or automated retarding of motions of the robot.

In another example, a motion- or gesture-controlled user interface for controlling a moving or movable part of the robot may be provided on the basis of retrieved used data, such as user presence or user identity and/or user proximity.

The wearable device, such as the smart glove, may comprise a sensor or sensors that are configured to detect gestures or positions of the user's hand, such as clenching a hand, pinching fingers, pointing with a finger or fingers and/or clasping hands.

The motion or gesture control may involve synchronized motion between a body part carrying the wearable device and a moving or movable part of the robot. For example, a movable part of the robot may go up or down on the basis of detected gestures or motions of the wearable device.

Moving or movable parts of an automated liquid handling robot may include one or more of the following: one or more grippers for gripping and transferring tip racks or tip boxes or other equipment and for releasing them at a suitable location such as onto a platform of the robot or in the proximity of the robot; and a dispensing head that is configured to move tips in a three-dimensional space, to aspirate liquid into the tips and to dispense the liquid from the tips into receptacles, such as microplate wells.

In an example, the motion or gesture control may be utilized in timing or triggering of actions in the robot, such as triggering a picking movement or action to make the robot pick tips to a dispensing head or triggering an aspirating or dispensing action.

In an example, the motion controlled user interface may be utilized for teaching movements to a liquid dispensing robot.

### Example 2: A smart watch

The wearable device may be implemented as a smart watch. In this case the laboratory device may be a hand-held electronic pipette or other electronic hand-held laboratory device.

In an embodiment, the entire use interface (UI) of a pipette is provided by a smart watch, which UI is used for communicating pipetting parameters or instructions to the pipette via an application or app in the wearable device. At the same time, the watch identifies the user and may save the user identifier (ID) to a log file. If the watch is removed from the user's wrist, the watch detects the removal via absence or non-detection of the user's skin contact, or pulse, and logs out of the app. Logging in again becomes possible only when a skin contact or a pulse is detected.

In an embodiment, the user wears the smart watch in one hand and operates a hand-held pipette with the other hand. When dispensing volumes are small and the microplate wells accordingly small and densely arranged, any pressing of pipette buttons may impair accurate positioning of the tip above a well into which liquid is to be dispensed. In such a case it is advantageous to use gesture control by means of moving the hand or turning the wrist that is holding the smart watch to actuate dispensing of liquid from the tip into the well. In this way the hand holding the pipette may be kept stationary. Preferably, all manual pressing actions of pipette buttons may be replaced by such gesture control during a pipetting event, which typically comprises aspirating and dispensing liquid and positioning the tip into or above a well.

### Example 3: A smart ring

The wearable device may be implemented as a smart ring worn around a user's finger.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### INDUSTRIAL APPLICABILITY

The present invention may be industrially applicable at least in control of electronic hand-held liquid handling devices or automated liquid handling robots.

## Claims

1. A method, comprising:
- providing a wearable electronic device that is configured to be worn by a user of a laboratory device;
- by means of the wearable device, retrieving user data that is specific to said user; and
- on the basis of the retrieved user data, providing a functionality for control of the laboratory device.

2. The method according to any of the preceding claims, wherein said wearable electronic device is configured to be worn around a body part of the user, and preferably said wearable electronic device comprises a smart watch, a smart bracelet, a smart ring, a smart necklace or smart glasses.

3. The method according to any of the preceding claims, wherein said laboratory device is a liquid handling or liquid dosing device, such as a hand-held electronic pipette or an automated liquid handling or dosing device.

4. The method according to any of the preceding claims, wherein said laboratory device is located in a sterile environment, such as in a sterile laminar flow hood or a sterile room.

5. The method according to any of the preceding claims, wherein the laboratory device does not comprise any physical buttons and preferably is sterilisable in its entirety.

6. The method according to any of the preceding claims, wherein the wearable electronic device and the laboratory device are connected to each other by a wireless connection.

7. The method according to any of the preceding claims, wherein said user data comprises user identity data or usage history data specific to said user or user location data, such as distance of the wearable device worn by the user from the laboratory device.

8. The method according to any of the preceding claims, wherein said functionality is executable either manually, for example by selecting or activating, by the user or automatically by the laboratory device.

9. The method according to claim 8, wherein said executable functionality is provided for use or execution either in the wearable electronic device and/or in the laboratory device.

10. The method according to claim 8 or claim 9, wherein
said executable functionality is provided in the laboratory device, and
said functionality comprises automated locking or automated unlocking of the laboratory device.

11. The method according to any of the preceding claims, wherein said functionality, such as locking of the laboratory device, is executed automatically when said user data satisfies a pre-determined criterion, such as when the distance between the user and the laboratory device is less than a pre-determined minimum distance.

12. The method according to any of the preceding claims, wherein
said executable functionality is provided in the wearable electronic device, and
said functionality comprises remote control of at least one function of the laboratory device, and preferably
said remote control of the laboratory device is provided in the wearable electronic device via a user interface in the wearable electronic device.

13. The method according to any of the preceding claims, wherein
said executable functionality is provided in the wearable electronic device, and
said functionality comprises providing a user-customized user interface of the laboratory device, and preferably
said user-customized user interface of the laboratory device is provided in the wearable device via input and/or output means, such as a display and/or buttons of the wearable electronic device.

14. The method according to claim 12 or claim 13, wherein said user interface or said user-customized user interface of the laboratory device is a user motion controlled user interface of the laboratory device.

15. The method according to any of the preceding claims, wherein if no user data can be retrieved, no functionalities for control of the laboratory device are provided, and preferably use of the laboratory device is disabled fully or partially, for example by locking the laboratory device or at least one or more of its operational modes.

16. The method according to any of the preceding claims, comprising
- retrieving environmental status data that is specific to the laboratory device, such as temperature data, air humidity data, fleet management data, or data related to an atmosphere; and
- on the basis of the retrieved user data and the retrieved environmental status data, providing a functionality for control of the laboratory device.

17. A system, comprising a wearable electronic device and a laboratory device connected to each other by a wireless connection, wherein said system is configured to be used in a method according to any of claims 1 to 16.

18. Use of a wearable electronic device, such as a smart watch, for providing user data which is specific to a user of a laboratory device, wherein on the basis of said user data, a functionality for control of the laboratory device is provided.

19. A wearable electronic device, which is connectable to a laboratory device and configured to be worn by a user of the laboratory device, the wearable electronic device comprising:
- means for retrieving user data that is specific to said user; and
- means for providing, on the basis of the retrieved user data, a user-specific user interface including at least one functionality for control of the laboratory device.
